# EUROPÄISCHE PATENTSCHRIFT

(11) **EP 3 181 174 B1**
(45) Veröffentlichungstag und Bekanntmachung des Hinweises auf die Patenterteilung: **08.01.2020**
(21) Anmeldenummer: 17155101.3
(22) Anmeldetag: 14.03.2008
(51) Int. Cl.: A61M 16/00, A61M 16/12, A61M 16/14, A61M 16/08

(54) **GASSTROMUMKEHRELEMENT**
GAS FLOW REVERSING ELEMENT
ÉLÉMENT D'INVERSION DE FLUX DE GAZ

(30) Priorität: 16.03.2007 DE 102007013385
(43) Veröffentlichungstag der Anmeldung: 21.06.2017
(62) Teilanmeldung aus: 08735419.7
(73) Patentinhaber: Ventinova Technologies B.V., 5612 AR Eindhoven (NL)
(72) Erfinder: Enk, Dietmar, 48653 Coesfeld (DE)
(74) Vertreter: Rössler, Matthias

(56) Entgegenhaltungen:
- DE-U1- 20 213 420
- FR-A- 1 499 477
- FR-A- 2 287 247
- US-A- 3 581 742
- US-A- 5 271 388
- US-A1- 2004 154 617

## Beschreibung

Der Gegenstand der vorliegenden Erfindung bezieht sich auf ein Gasstromumkehrelement und ein Verfahren zur wahlweisen Erzeugung eines Gasstromes von oder zu einem Leitungsanschluss zum Gasaustausch in abgeschlossenen oder nur teilbelüfteten Räumen. Eine Anwendung ist der Anschluss eines solchen Gasstromumkehrelementes an einen in den Atemweg eines Patienten einbringbaren Katheter oder an eine Kanüle.

Bei der Beatmung eines Patienten wird normalerweise eine Maske oder ein Tubus eingesetzt, über die bzw. den dem nach außen abgedichteten Atemweg mit niedrigem Druck ein Gas oder Gasgemisch, insbesondere Sauerstoff und Luft, zugeführt wird. Alternativ kann man ein solches Gas oder Gasgemisch aber auch mit hohem Druck und hohem Fluss stoßweise durch einen dünnen, ungeblockten Katheter in den nach außen offenen Atemweg injizieren (sogenannte Jet-Ventilation). Dieses Verfahren wird heute insbesondere bei diagnostischen und therapeutischen Eingriffen im Bereich des oberen Atemweges bzw. der Lunge eingesetzt. Mittels dieses Verfahrens kann ein Patient aber auch durch einen durch die Haut direkt in die Luftröhre eingebrachten Katheter bzw. eine so platzierte Kanüle mit Sauerstoff versorgt werden (sogenannte transtracheale Jet-Ventilation). Dieses spezielle Verfahren ist Bestandteil der heute gültigen Algorithmen zum Management eines schwierigen Atemweges und insbesondere der Situation, in der ein Patient konventionell nicht zu beatmen bzw. zu intubieren ist (sogenannte "cannot ventilate, cannot intubate"-Situation).

Unter ungünstigen Bedingungen kann aber gerade die transtracheale Jet-Ventilation auch potentiell lebensgefährlich sein kann. Ist der Atemweg eines Patienten weitgehend oder sogar vollständig z.B. durch Schwellung oder Blutung verlegt, wird die Lunge des Patienten durch die Injektion von Sauerstoff mehr und mehr gebläht. Dabei droht eine Lungenzerreißung (sogenanntes Barotrauma). Weiterhin kann die Drucksteigerung im Brustkorb zu einer für den Patienten nicht minder gefährlichen Kreislaufstörung bzw. zu einem Kreislaufzusammenbruch führen, da das Blut nicht mehr in ausreichendem Maße zum Herzen zurückfließen kann.

Seit einiger Zeit ist der "Oxygen Flow Modulator" bekannt, der in der beschriebenen kritischen Situation zum bidirektionalen Luftweg wird. Dieser wird beispielsweise in einem 2004 herausgegebenen Prospekt "Products For The Difficult Airway" der Firma Cook Medical Incorporated auf Seite 10 beschrieben.

Durch ein intermittierendes Schließen und Freigeben mehrerer Öffnungen kann man mit diesem Instrument nicht nur sehr einfach den Sauerstofffluss zum Patienten kontrollieren, sondern auch den Druck im Brustkorb entlasten. Mit diesem Instrument ist es möglich, selbst bei einem komplett verlegten oberen Atemweg durch einen in die Luftröhre eingebrachten Katheter bzw. eine eingestochene Kanüle die Sauerstoffversorgung eines Patienten zumindest für kurze Zeit sicherzustellen.

Die ca. 3,5 Liter Atemluft, die in dieser Situation bei mittlerer Volumendehnbarkeit des Thorax und normalen Atemwegswiderständen pro Minute passiv, also lediglich durch den leichten Überdruck im Brustkorb, durch einen Katheter mit einem Innendurchmesser von 2,0 mm über das Instrument auch wieder aus der geblähten Lunge abströmen können, sind aber für eine ausreichende Beatmung eines Erwachsenen zu wenig (sogenannte Hypoventilation). Es kommt zur Retention von Kohlendioxid und in der Folge entwickelt sich schnell eine unerwünschte, zunehmende Übersäuerung des Blutes (sogenannte respiratorische Azidose).

Der "Oxygen Flow Modulator" leistet dabei deutlich mehr als herkömmliche Mittel, mit denen man den Patienten lediglich "aufblasen" kann. Das Problem, dass man erwachsene Patienten durch einen Katheter oder eine Kanüle mit geringem Querschnitt nicht ausreichend beatmen kann, löst er aber nicht. Neben den erwähnten Risiken hat insbesondere diese Limitierung in jüngster Zeit zu einer Diskussion über den Stellenwert der notfallmäßigen Jet-Ventilation in Fachjournalen geführt.

DE 20213420 U1 beschreibt ein Beatmungsgerät, insbesondere zur notfallmäßigen transtrachealen Beatmung, mit einer steuerbaren Druckgasquelle, einem verzweigten Rohr zur Zu- und Abfuhr des Atemgases zum bzw. vom Patienten, wobei eine Venturi-Düse zur Unterstützung der Exspiration genutzt werden soll. Dieses Beatmungsgerät ist eher wie ein Absauggerät für Lungensekret und dergleichen konstruiert, wobei es auf hohen Saugdruck, nicht jedoch auf möglichst große Volumenströme bei der Absaugung ankommt. Laut Fachliteratur kann man mit diesem Beatmungsgerät durch einen Katheter mit einem Innendurchmesser von 2,0 mm das Atemminutenvolumen um ca. 1 Liter / Minute steigern. Das so resultierende Atemminutenvolumen ist aber für eine Normoventilation eines Erwachsenen in der Regel immer noch nicht ausreichend. Die Konstruktion sieht zudem keine Möglichkeit vor, um mit einer Hand den in Richtung Patient wirksam werdenden Über- oder Unterdruck zu kontrollieren.

Die US 5 271 388 A offenbart einen Oszillationsventilator, bei dem ein Gasstrom hin und weg vom Patienten durch eine rotierende Düse realisiert wird.

Aus der US 3 581 742 A ist ein Hochdruckbeatmungsgerät bekannt, durch das Luft auch gegen starke Widerstände dem Patienten zugeführt werden kann.

Aus der US 2004/154617 A1 ist eine Verneblungsvorrichtung bekannt, die zum Zuführen von Medikamenten oder Ähnlichem eingesetzt wird.

Aus der FR 2 287 247 A ist eine Vorrichtung zur Anzeige einer Atmungsaktivität eines Patienten bekannt.

FR1 499 477 A ist auf eine Vorrichtung gerichtet, bei der ein Patient über eine Maske beatmet wird. Dabei soll die Vorrichtung sich an den Atem-Rhythmus des Patienten automatisch anpassen.

Hiervon ausgehend liegt der vorliegenden Erfindung die Zielsetzung zugrunde, eine einfache Vorrichtung und ein Verfahren zu schaffen, mit denen einerseits Sauerstoff der Lunge zugeführt, andererseits aber auch Kohlendioxid bzw. verbrauchte Atemluft in ausreichendem Maße aktiv aus der Lunge herausgeführt werden kann.

Diese Zielsetzung wird durch ein Gasstromumkehrelement mit den Merkmalen des Anspruchs 1 erreicht. Vorteilhafte Weiterbildungen und Ausgestaltungen des Gasstromumkehrelementes und des Verfahrens sind Gegenstand der jeweils abhängigen Ansprüche.

Das erfindungsgemäße Gasstromumkehrelement dient zur Ausnutzung eines unter Überdruck stehenden Gasvorrates, insbesondere Sauerstoff, zur wahlweisen Erzeugung eines Gasstromes von oder zu einem Leitungsanschluss, insbesondere zum Anschluss an einen in den Atemweg eines Patienten einbringbaren Katheter oder einer Kanüle. Es ist ausgebildet als ein Hauptstück mit Abzweigungsstück, wobei das Hauptstück einen Druckanschluss zum Anschluss an den Gasvorrat mit mindestens einer verschließbaren Auslassöffnung verbindet und das Abzweigungsstück von dem Hauptstück zu einem Leitungsanschluss führt. Dabei ist in dem Hauptstück eine Düse, insbesondere eine Injektor-Düse, so ausgebildet und angeordnet, dass durch einen von dem Druckanschluss durch die Düse zu der Auslassöffnung strömenden Gasstrom auch in dem Abzweigungsstück ein Gasstrom in Richtung der Auslassöffnung erzeugbar ist, wobei das Hauptstück vom Abzweigungsstück bis zu der Auslassöffnung einen im Wesentlichen gleichbleibenden oder in Richtung Auslassöffnung zunehmenden Strömungsquerschnitt aufweist. Es kann grundsätzlich für die Düse jedes Konstruktionsprinzip gewählt werden, das mittels eines Gasstromes eine Saugwirkung erzeugt.

Bevorzugt findet hier aber das Prinzip einer Gasstrahlpumpe Anwendung. Insbesondere mit Blick auf das Absaugen größerer Gasvolumina ist es dabei wichtig, möglichst auf eine Querschnittsverengung im Hauptstück zwischen dem Abzweigungsstück und der Auslassöffnung zu verzichten.

Es gibt verschiedene Möglichkeiten, eine gute Funktion des Gasstromumkehrelementes zu erreichen. Es hat sich in Versuchen gezeigt, dass es Dimensionierungen von Düse und Mischkanal gibt, die bei Unterschallgeschwindigkeit gute Ergebnisse zeigen, aber auch Dimensionierungen für eine Düsenaustrittsgeschwindigkeit im Überschallbereich können eingesetzt werden.

Experimente haben gezeigt, dass sich bei einer Ausführungsform der Erfindung die Ausbreitung des Gasstroms nach Austritt aus der Düse in einen Mischkanal in drei charakteristische Bereiche gliedern lässt, den Aufweitungsbereich, den Verwirbelungsbereich und den Bereich gleichförmiger Bewegung. Der Aufweitungsbereich beginnt unmittelbar am Auslass der Düse. An dieser Stelle ist der Luftstrom nicht in der Lage, der scharfen Kante der Düse zu folgen und weitet sich über eine Länge, die dem ca. 3- bis 5-fachen des Durchmessers der Düse entspricht, auf. An den Aufweitungsbereich schließt der Verwirbelungsbereich an, in dem sich eine turbulente Strömung im Gasstrom ausbildet. In diesem Bereich wird das einströmende Gas mit dem im Hauptstück befindlichen Gas vermischt und gemeinsam mit diesem weiter in Richtung der Auslassöffnung transportiert. Der Verwirbelungsbereich bildet sich in der Regel über eine Länge, die dem ca. 5- bis 7-fachen des Durchmessers der Düse entspricht aus. An den Verwirbelungsbereich schließt der Bereich gleichförmiger Bewegung an, in dem das einströmende Gas mit dem zuvor im Hauptstück befindlichen Gas homogen vermischt ist. Hier treten nahezu keine turbulenten Strömungen mehr auf, sondern das Gas oder Gasgemisch bewegt sich gleichförmig in Richtung der Auslassöffnung. Somit sollte der Mischkanal zumindest eine Länge, die dem 8-fachen des Düsendurchmessers entspricht, aufweisen. Bevorzugt sind Längen des Mischkanals, die dem 5- bis 30-fachen des Durchmessers der Düse entsprechen.

Im Durchmesser sind das Lumen des Abzweigungsstückes und des Hauptstückes zwischen Düse und Auslassöffnung sinnvollerweise nicht kleiner als das Lumen des in den Atemweg eines Patienten einbringbaren Katheters oder der Kanüle (z.B. mindestens 2 mm bei Verwendung eines Katheters mit 2 mm Innendurchmesser), da sonst insbesondere für das Abfließen bzw. Ansaugen des Gases aus der Lunge störender, zusätzlicher Widerstand geschaffen wird. Eine deutlich größere Öffnung bzw. Öffnungen zum Abzweigungsstück führen wieder zu dem bautechnischen Problem, diese im bzw. am kleineren Lumen des Hauptstückes unterzubringen. Zudem ist dies wie auch ein deutlich größeres Lumen des Abzweigungsstückes ohne relevanten Effekt auf den Durchflusswiderstand, der dann hauptsächlich durch den in den Atemweg eines Patienten einbringbaren Katheter oder der Kanüle bestimmt wird.

Die beschriebene Ausführung erlaubt es nicht nur, ein Gas oder Gasgemisch unter Überdruck einem Katheter oder einer Kanüle zuzuführen und damit dort einen Gasstrom in Richtung Patient zu bewirken, sondern ermöglicht es auch, einen Gas- oder Gasgemischstrom zur Erzeugung von Unterdruck und damit einen Gasstrom in einem Katheter oder einer Kanüle vom Patienten weg zu nutzen. Dies kann durch Öffnen und Schließen der Auslassöffnung wechselweise geschehen, obwohl das Gasstromumkehrelement nur mit Überdruck versorgt wird.

Zur Sicherheit des Patienten ist es wichtig, dass nicht schon bei einer versehentlichen Manipulation am Gasstromumkehrelement irgendein Einfluss auf den Atemweg ausgeübt wird. Daher ist es vorteilhaft, wenn vorzugsweise in dem Abzweigungsstück mindestens eine Sicherheitsöffnung vorhanden ist. Bevorzugt sollten sogar zwei, besonders bevorzugt zwei nebeneinander oder einander gegenüberliegende Sicherheitsöffnungen vorhanden sein. Dabei dienen diese Sicherheitsöffnungen gleichzeitig der Steuerung des Über- oder Unterdruckes in Richtung Patient. Bei Freigabe wirken sie zudem als Druckausgleichsöffnungen und ermöglichen den Ausgleich eines intrathorakalen Über- oder Unterdruckes, wenn man die Jet-Beatmung kurz pausiert. Dadurch kann sich der Druck in der Lunge mit dem Außendruck äquilibrieren. Dies ist insbesondere dann wichtig, wenn man einen komplett verlegten Atemweg hat, und man nicht weiß, ob die Lunge des Patienten aktuell etwas zu stark aufgeblasen oder leer gesaugt ist.

Bei Verbinden des Druckanschlusses des Gasstromumkehrelementes mit einer Druckgasquelle strömt Gas durch die Düse zur Auslassöffnung, was ohne Sicherheitsöffnungen direkt zu einem Unterdruck am Leitungsanschluss und damit eventuell auch im Atemweg führt. Durch die noch offenen Sicherheitsöffnungen, die dazu einen genügend großen Querschnitt haben sollten, wird dies vermieden, da der Unterdruck durch dort einströmende Umgebungsluft ausgeglichen wird. Erst wenn alle Sicherheitsöffnungen gezielt, z.B. durch die Finger einer Bedienperson, geschlossen werden, entsteht am Leitungsanschluss ein Unterdruck.

Durch zusätzliches Schließen der Auslassöffnung kann dann ein Gas oder Gasgemisch auch in den Atemweg geleitet werden. Bei Freigabe der Sicherheitsöffnungen entweicht das Gas oder Gasgemisch hingegen sofort quantitativ in die Umgebungsluft. Durch wechselweises Öffnen und Schließen der Auslassöffnung bei geschlossenen Sicherheitsöffnungen erreicht man eine wirkungsvolle Beatmung. Bevorzugt ist die Auslassöffnung als sich nach außen öffnender Trichter geformt, der nicht so leicht versehentlich verschlossen werden kann wie eine kleine Auslassöffnung.

Besonders bei einer Notfallversorgung ist es vorteilhaft, nach einer bevorzugten Ausführung der Erfindung die Auslassöffnung und die Sicherheitsöffnungen so anzuordnen, dass sie manuell, vorzugsweise mit Handballen und/oder Fingern nur einer Hand eines Helfers oder einer Bedienperson wahlweise einzeln oder gemeinsam ganz oder teilweise verschließbar sind. So kann ein Helfer mit einer Hand die Beatmung nach Bedarf durchführen, unterbrechen oder durch teilweises Verschließen der Öffnungen regulieren, hat aber noch die andere Hand für weitere Maßnahmen oder Tätigkeiten (z.B. Sicherung des Katheters oder der Kanüle) frei.

Bevorzugt ist der Druckanschluss des Gasstromumkehrelementes zur Herstellung einer Verbindung mit einer Druckgasquelle, vorzugsweise einer Sauerstoffdruckflasche, ausgebildet. In den meisten Notarztwagen oder Unfallstationen steht zumindest eine kleine Sauerstoffdruckflasche (z.B. 2 Liter) zur Verfügung, mit der das Gasstromumkehrelement über einen Zeitraum von wenigstens 20 Minuten betrieben werden kann.

Es kann erforderlich sein, dem Gas oder Gasgemisch Medikamente beizumischen. Dazu kann besonders vorteilhaft ein verschließbarer Seitenzugang an dem Abzweigungsstück angeordnet sein, vorzugsweise zwischen den Sicherheitsöffnungen und dem Leitungsanschluss. So können z.B. Adrenalin, Lokalanästhetika, Sekretolytika und dergleichen mittels des schnell fließenden Gases oder Gasgemisches zum Patienten hin fein verdüst werden. Damit kann ein Medikament effektiv und großflächig in den Bronchien verteilt werden und wird so quantitativ schneller resorbiert. Zudem bietet der Seitenzugang eine Anschlussmöglichkeit für eine Kapnometrie-Leitung, über die aus der abgesaugten Atemluft im Nebenstrom eine kleine Probe für die Messung der Kohlendioxidkonzentration abgeführt werden kann. So lässt sich die Effektivität der Beatmung im Verlauf abschätzen (über die Zeit ab- bzw. zunehmende Ventilation). Vorstellbar ist auch der direkte oder indirekte Anschluss einer Gasfluss- oder Druckmesseinrichtung.

Erfindungsgemäß bilden bevorzugt das Hauptstück und das Abzweigungsstück zusammen etwa ein T-Stück mit dem Hauptstück als Querbalken. Dies macht den Aufbau besonders übersichtlich und die Düsenkonstruktion sehr einfach.

Alternativ wird erfindungsgemäß ein Gasstromumkehrelement vorgeschlagen, bei dem das Hauptstück und das Abzweigungsstück zusammen etwa ein Y-Stück bilden, wobei die Auslassöffnung den Fuß des Y bildet. Auch eine Anordnung, bei der das Abzweigungsstück einen Winkel zwischen 10° und 90° zu einem geraden Hauptstück bildet, kann für die Strömungsführung vorteilhaft sein.

Zur Vereinfachung der Herstellung ist das Gasstromumkehrelement erfindungsgemäß bevorzugt aus mehreren Teilelementen zusammensetzt, insbesondere zusammengeschraubt. Dies erlaubt die Verwendung von Standardbauteilen für einen Teil der Anordnung.

Ebenfalls bevorzugt sind der Druckanschluss und/oder der Leitungsanschluss und gegebenenfalls der Seitenzugang als sogenannter "Luer Lock" ausgeführt, um die druckfeste und druckdichte Verbindung mit Standardbauteilen zu erlauben.

Aus Sicherheitsgründen erscheint es sinnvoll, den Hochdruckteil des Gasstromumkehrelementes einschließlich des Verbindungsschlauchanschlusses zu kapseln.

Im Allgemeinen soll erfindungsgemäß das Gasstromumkehrelement im Wesentlichen aus Kunststoff gefertigt sein, wobei die Düse aus Kunststoff oder aus einem metallischen Einsatz besteht.

Im Folgenden soll noch einiges zu dem erfindungsgemäßen Verfahren zum Betrieb eines Gasstromumkehrelementes erläutert werden. Hauptsächlich besteht das Verfahren darin, dass ein im Wesentlichen konstanter Gasdruck, insbesondere Sauerstoffdruck, an den Druckanschluss angelegt wird, wobei zur Erzeugung von Überdruck am Leitungsanschluss die Auslassöffnung und gegebenenfalls alle Sicherheitsöffnungen verschlossen werden und zur Erzeugung von Unterdruck die Auslassöffnung bei gegebenenfalls weiter geschlossenen Sicherheitsöffnungen geöffnet wird.

Durch dieses Prinzip des Ansaugens von Atemluft über einen in den Atemweg eines Patienten einbringbaren Katheter oder einer Kanüle kann das Gasstromumkehrelement mit der herkömmlichen Jet-Ventilation sehr sinnvoll kombiniert werden. Nicht nur im Notfall kann ein Patient so durch Leitungen mit kleinem Querschnitt (z.B. transkutan in die Luftröhre eingebrachte Katheter oder Kanülen, aber auch sogenannte Tubuswechsler oder Arbeitskanäle von flexiblen Fiberoptiken) effektiv beatmet werden (d.h. ausreichende Versorgung mit Sauerstoff bei gleichzeitiger Elimination des anfallenden Kohlendioxids). Dabei ist es unerheblich, ob der obere Atemweg - wie bei konventioneller Jet-Ventilation unerlässlich - offen oder hingegen teilweise wenn nicht sogar vollständig verlegt ist.

Für den Einsatz des erfindungsgemäßen Gasstromumkehrelementes werden lediglich eine Sauerstoffdruckflasche und eine Verbindungsleitung benötigt. Das Gasstromumkehrelement kann so ausgeführt sein, dass es insbesondere auch für verschiedene Drücke der Sauerstoffquellen anpassbar ist bzw. konstruiert werden kann. Z.B. haben Sauerstoffquellen in den Vereinigten Staaten einen Entnahmedruck von 3,5 bar, in Europa hingegen von bis zu 5 bar.

Eine wichtige Größe bei dem Gasstromumkehrelement ist der Abstrahldruck an der Düse, durch den ein Unterdruck an dem Leitungsanschluss erzeugt wird. Der Abstrahldruck korreliert dabei direkt mit dem Fluss und der resultierenden Fließgeschwindigkeit des Gases bzw. Gasgemisches durch das Gasstromumkehrelement und die Fließgeschwindigkeit des Gases bzw. Gasgemisches wiederum mit dem Unterdruck am Leitungsanschluss. Mit Blick auf die gewünschte große Saugleistung muss man mit sehr hohen Gasgeschwindigkeiten arbeiten. Diese lassen sich bevorzugt durch Öffnungen der Injektor-Düse von 1 mm oder kleiner erreichen. Bei einem Druckgasfluss von 15 Litern/Minute durch eine solche Düse können sich Gasgeschwindigkeiten im Überschallbereich ergeben. Wichtig ist auch, dass der erzeugte Unterdruck im Abzweigungsstück in einen relativ hohen Gasstrom umgesetzt werden kann, was dann am besten gelingt, wenn keine den Gasstrom signifikant behindernden Querschnittsverengungen im Hauptstück zwischen Abzweigungsstück und Auslassöffnung vorhanden sind.

Das Gasstromumkehrelement kann auch mit einem vorgeschalteten Flussmeter kombiniert werden. Dadurch kann man sich sehr einfach und schnell an den Patienten anzupassen (z.B. werden so Kinder mit geringerem Sauerstofffluss, damit geringerem Abstrahldruck an der Düse und somit auch geringerem Unterdruck an dem Leitungsanschluss beatmet). Aus einer Reduktion des Gas(gemisch)flusses folgt immer eine weniger starke Beatmung (Reduktion des Atemminutenvolumens), aus einer Erhöhung des Gas(gemisch)flusses hingegen eine stärkere Beatmung (Erhöhung des Atemminutenvolumens).

Da ein typischerweise verwendeter Katheter bzw. eine Kanüle einen erheblichen Durchflusswiderstand bietet, wird der am Leitungsanschluss wechselweise wirksam werdende Überdruck bzw. Unterdruck gedämpft. Druckänderungen werden in der Lunge eines Patienten primär immer durch das nach normaler Ausatmung noch in der Lunge befindliche Gasvolumen (sogenannte funktionelle Residualkapazität), sekundär dann durch die elastische Thoraxwand und das verschiebliche Zwerchfell gedämpft. Dadurch wird die Gefahr eines Barotraumas minimiert. Ein Barotrauma ist überhaupt nur bei längerer Fehlbedienung des Gasstromumkehrelementes durch den Anwender möglich. Dem Risiko kollabierender Lungenabschnitte (Entstehung sogenannter Atelektasen) bei einem zu langen und starken Ansaugen von Atemluft aus der Lunge steht hingegen ein gerade in Notfallsituationen gewünschter positiver Kreislaufeffekt gegenüber, verbessert doch ein niedriger Druck im Brustkorb den Blutrückfluss zum Herzen.

Bei Ventilationstests in einem Lungenmodell wurden mit einem Sauerstofffluss von 15 Litern / Minute und einem Überdruck über Umgebungsdruck von ca. 1 bar unter Verwendung eines ca. 10 cm langen, transtrachealen Katheters mit 2 mm Innendurchmesser ein für die Beatmung eines Erwachsenen ausreichendes Atemminutenvolumen von mehr als 6 Litern / Minute gemessen. Dadurch ergibt sich zwischen dem Volumenstroms durch das Abzweigungsstück und dem Volumenstrom durch den Druckanschluss ein Verhältnis von mindestens 0,4 : 1, vorzugsweise 0,6 : 1 bis 2 : 1. Hier ist noch anzumerken, dass bei Jet-Ventilation durch einen transtrachealen Katheter anatomischer Totraum wegfällt und daher grundsätzlich bereits ein kleineres Atemminutenvolumen für eine ausreichende Beatmung (sogenannte Normoventilation) eines Patienten ausreicht.

Bemerkenswert war bei den Versuchen eine mehr oder weniger physiologische Relation zwischen Inspirations- und Exspirationszeit (hier: Zeit der Injektion von Sauerstoff und Zeit des Absaugens von Atemluft). Dies minimiert beatmungsbedingte Kreislaufprobleme. Bei passivem Abstrom von Atemluft durch einen solchen transtrachealen Katheter ergibt sich hingegen ein groteskes Inspirations-/Exspirationsverhältnis mit einer zur Inspirationszeit vielfach längeren Exspirationszeit.

Die Limitierung des Unterdruckes gegenüber dem Umgebungsdruck (relativer Druck) auf etwa -0,3 bar, der bei Versuchen erreicht wurde, ist vorteilhaft: Liegt der Katheter bzw. die Kanüle der Schleimhaut an, kann er bzw. sie sich nicht zu stark ansaugen und dadurch die Schleimhaut lokal schädigen. Prinzipbedingt wird der Katheter bzw. die Kanüle zudem bei jeder Injektion von der Schleimhaut weggedrückt.

Die simple, aber gut funktionierende und für den Patienten und Anwender nahezu gefahrlose "Wechselschaltung" zwischen Über- und Unterdruck mit zyklischer Fluss- und Druckumkehr im Gasstromumkehrelement eröffnen neue Möglichkeiten insbesondere bei der Notfallbeatmung. Das Fehlen jeglicher Mechanik bzw. beweglicher Teile macht das relativ kleine Gerät nicht nur wartungsfrei, sondern auch leicht verständlich und in der Anwendung sicher. Etwas (unter sehr ungünstigen Umständen) potentiell Gefährliches kann immer nur durch eine bewusste Manipulation des Anwenders geschehen, nicht aber durch den Anschluss des Gasstromumkehrelementes als solches bzw. das Aufdrehen einer angeschlossenen Druckgasquelle.

Der Anwendungsbereich geht aber weit über die "cannot ventilate, cannot intubate"-Situation hinaus. Zweifellos kann auch ein Patient mit nur teilweise bzw. zeitweilig verlegtem oberen Atemweg von diesem Beatmungsprinzip profitieren, schafft doch die saugunterstützte Ausatmung einen virtuell größeren, freien Atemweg. Selbst bei Patienten mit offenem oberen Atemweg ist zu erwarten, dass es aufgrund effektiveren Gasaustausches bzw. größeren Gasumsatzes in der Lunge zu einer schnelleren Sauerstoffanreicherung bei gleichzeitig effektiverer Kohlendioxidelimination kommt.

Auch die Möglichkeit einer einfachen Jet-Ventilation besteht: Hierfür müssen lediglich die Auslassöffnung und die Sicherheitsöffnungen zeitgleich verschlossen und wieder frei gegeben werden, d.h. die saugunterstützte Ausatmung ist lediglich eine Option, die man nutzen kann, aber nicht nutzen muss.

Wünschenswert ist die Implementierung dieses Prinzips in konventionelle Jet-Ventilatoren. Die heutigen Geräte erfordern alle einen ausreichend großen, freien oberen Atemweg des Patienten. Im Falle einer weitgehenden bzw. vollständigen Verlegung des Atemweges (z.B. durch Instrumentation) stoppen diese Geräte, sobald ein zu hoher Druck im Atemweg gemessen wird, mit der Folge, dass der Patient nicht mehr beatmet wird. Basierend auf der vorliegenden Erfindung lassen sich Jet-Ventilatoren bauen, die unabhängig vom freien Atemwegsquerschnitt und sogar bei vollständiger Verlegung des Atemweges einen Patienten nicht nur ausreichend, sondern sehr sicher und effektiv beatmen können.

Ausgehend vom Funktionsprinzip des Gasstromumkehrelementes könnte man mittels einer sich über der Auslassöffnung drehenden Lochscheibe bzw. einem Lochrad eine sehr präzise Steuerung des Inspirations-/Exspirationsverhältnisses sowie der Beatmungsfrequenz realisieren. Durch geeignete Ausschnitte in dieser Lochscheibe bzw. in diesem Lochrad wären Inspirations- und Exspirationszeit auch so festzulegen, dass das injizierte Gas(gemisch)volumen nachfolgend wieder abgesaugt wird. Mit einer sich dann schnell drehenden Lochscheibe bzw. einem Lochrad ließen sich zudem vergleichsweise einfach Beatmungsfrequenzen im Sinne einer Hochfrequenzventilation (z.B. zur lungenprotektiven Beatmung kritisch kranker Patienten) realisieren, die in herkömmlichen Geräten technisch erheblich höheren Aufwand erfordern. Dies würde völlig neue Möglichkeiten bei diagnostischen und therapeutischen Interventionen eröffnen.

Ausführungsbeispiele der Erfindung, auf die diese jedoch nicht beschränkt ist, werden im Folgenden anhand der Zeichnung näher erläutert und zwar zeigen:
Fig. 1 einen Längsschnitt durch ein erfindungsgemäßes Gasstromumkehrelement mit schematisch dargestellten Peripheriegeräten,
Fig. 2 und 3 alternative Ausgestaltungen von Düse und Hauptstück,
Fig. 4 schematisch die Größenverhältnisse einzelner Teile des Gasstromumkehrelementes und
Fig. 5 ein Gasstromumkehrelement mit ergonomisch geformtem Gehäuse.

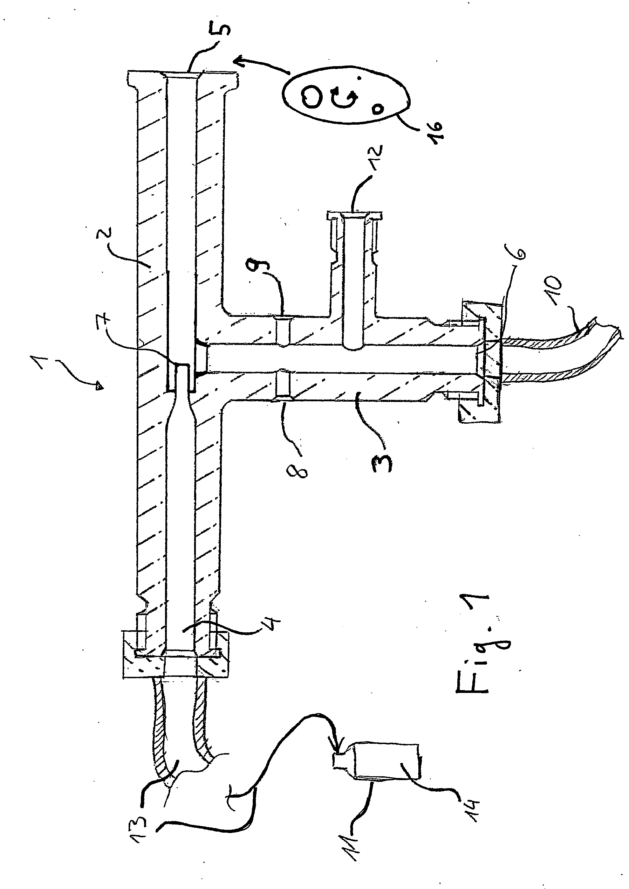

Fig. 1 zeigt ein Gasstromumkehrelement 1 mit einem Hauptstück 2, welches einen Druckanschluss 4 mit einer Auslassöffnung 5 verbindet. Der Druckanschluss 4 kann über eine Verbindungsleitung 13 mit einem unter Überdruck stehenden Gasvorrat 14 in einer Druckgasquelle 11 verbunden werden. Im Allgemeinen steht für die Notfallversorgung von Patienten eine Sauerstoffdruckflasche zur Verfügung. Von dem Hauptstück 2 zweigt ein Abzweigungsstück 3 ab, welches zu einem Leitungsanschluss 6 führt. In dem Hauptstück 2 ist eine Düse 7 ausgebildet, durch die Gas vom Druckanschluss 4 zur Auslassöffnung 5 strömen kann, wobei diese Düse 7 in der Nähe des Abzweigungsstückes 3 liegt, so dass das durch die Düse 7 zur Auslassöffnung 5 strömende Gas einen Unterdruck im Abzweigungsstück 3 erzeugt. Hier findet das Prinzip einer Gasstrahlpumpe Anwendung. Es kann jedoch jede Anordnung gewählt werden, die mittels eines Gasstromes eine Saugwirkung erzeugen kann. Dazu ist es insbesondere nicht erforderlich, dass das Abzweigungsstück 3, wie im Ausführungsbeispiel gezeigt, einen rechten Winkel α (Alpha) zu dem Hauptstück 2 bildet. Weiter weist das Abzweigungsstück 3 eine erste 8 und eine zweite 9 Sicherheitsöffnung auf. Solange diese beiden Sicherheitsöffnungen 8, 9 nicht gezielt verschlossen werden, übt die Vorrichtung auch bei strömendem Gas keinen Einfluss auf den Atemweg eines Patienten aus. Nur wenn diese Sicherheitsöffnungen 8, 9 verschlossen werden, entsteht im Abzweigungsstück 3 ein Unterdruck, solange die Auslassöffnung 5 offen ist, und ein Überdruck, wenn die Auslassöffnung 5 verschlossen wird. Bevorzugt sollten alle Öffnungen so angeordnet sein, dass sie von einer Bedienperson zwar mit einer Hand bewusst, aber kaum versehentlich verschlossen werden können. Zumindest die Auslassöffnung 5 sollte dann mit einem Finger verschließbar sein, da sie bei der Beatmung wechselweise geöffnet und geschlossen werden muss. Um auch hier eine versehentliche Betätigung möglichst zu vermeiden, kann das Hauptstück 2 vor der Auslassöffnung 5 trichterförmig erweitert sein. Wie schematisch angedeutet, kann vor der Auslassöffnung 5 auch eine drehbare Lochscheibe 16 mit unterschiedlich großen Löchern angeordnet sein, die dann von einer Bedienperson in die jeweils gewünschte Stellung gedreht werden kann. Das Abzweigungsstück 3 weist außerdem einen verschließbaren Seitenzugang 12 auf, durch den Medikamente zugegeben oder Sonden eingeführt werden können. An den Leitungsanschluss 6 kann ein Katheter 10 oder eine Kanüle angeschlossen werden. Bevorzugt ist jeder der Anschlüsse als sogenannter "Luer Lock" ausgebildet. Die ganze Anordnung ist vorzugsweise aus Kunststoff hergestellt, wobei die Düse 7 je nach Bedarf auch als metallisches Teil eingesetzt sein kann.

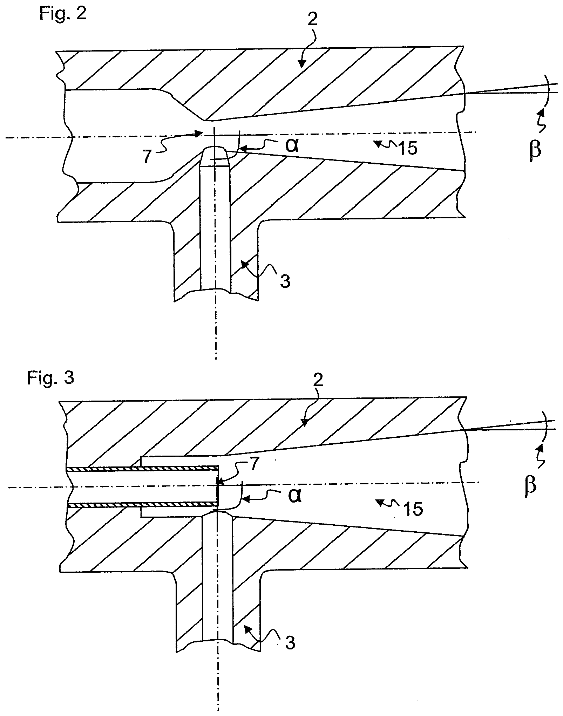

In den Figuren 2 und 3 sind alternative Ausgestaltungen des Hauptstückes 2 und der Düse 7 dargestellt. Fig. 2 zeigt eine Ausgestaltung des Hauptstückes 2 als Venturi-Düse, bei der an der Stelle der höchsten Geschwindigkeit und damit des geringsten Druckes das Abzweigungsstück 3 einmündet. Bei beiden Fig. 2 und 3 bildet das Hauptstück 2 hinter dem Abzweigungsstück 3 einen sich konisch mit einem Öffnungswinkel β (Beta), von z.B. 1° bis 7°, erweiternden Kanal 15.

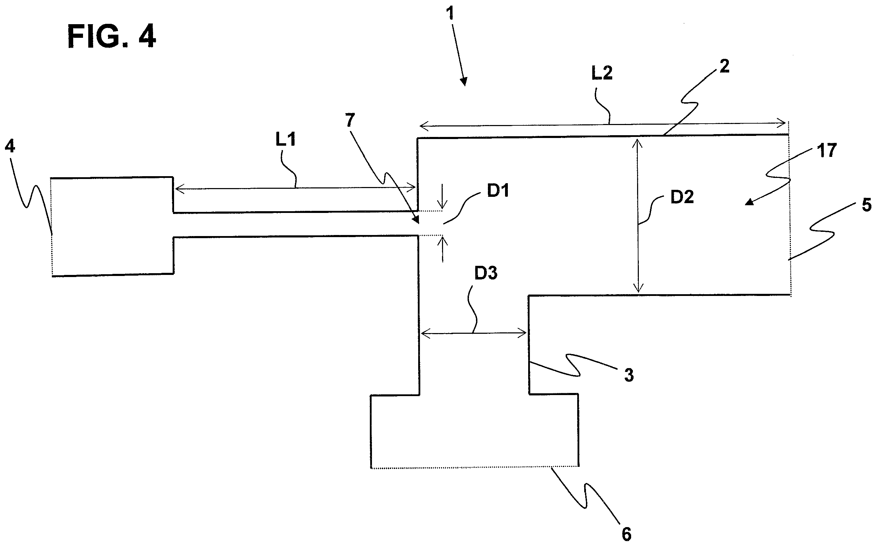

Figur 4 zeigt eine weitere schematische Darstellung einer Ausführungsform des Gasstromumkehrelements 1 mit Hauptstück 2 und Abzweigungsstück 3. Dabei bezeichnet D1 den Innendurchmesser der Düse 7, D2 den Innendurchmesser des Mischkanals 17 und D3 den Innendurchmesser des Abzweigungsstücks 3. L1 bezeichnet die Länge der Düse 7, L2 die Länge des Mischkanals 17. Bei einer bevorzugten funktionsfähigen Testanordnung war der Innendurchmesser D1 der Düse 7 zwischen 0,5 und 2 mm, vorzugsweise bei 1 mm, der Innendurchmesser D2 des Mischkanals 17 zwischen 1 und 5 mm, bevorzugt bei 2 bis 3 mm, insbesondere 2,5 mm. Der Innendurchmesser D3 des Abzweigungsstücks 3 lag in der gleichen Größenordnung wie der Innendurchmesser D2 des Mischkanals 17. Die Länge L1 der Düse 7 lag bei 5 bis 20 mm, vorzugsweise bei 15 mm. Die Länge L2 des Mischkanals betrug 10 bis 40 mm, bevorzugt 20 bis 30 mm, wobei das Verhältnis der Länge L2 des Mischkanals 17 und des Innendurchmessers D1 der Düse 7 zwischen 2:1 und 100:1, bevorzugt zwischen 5:1 und 50:1 lag.

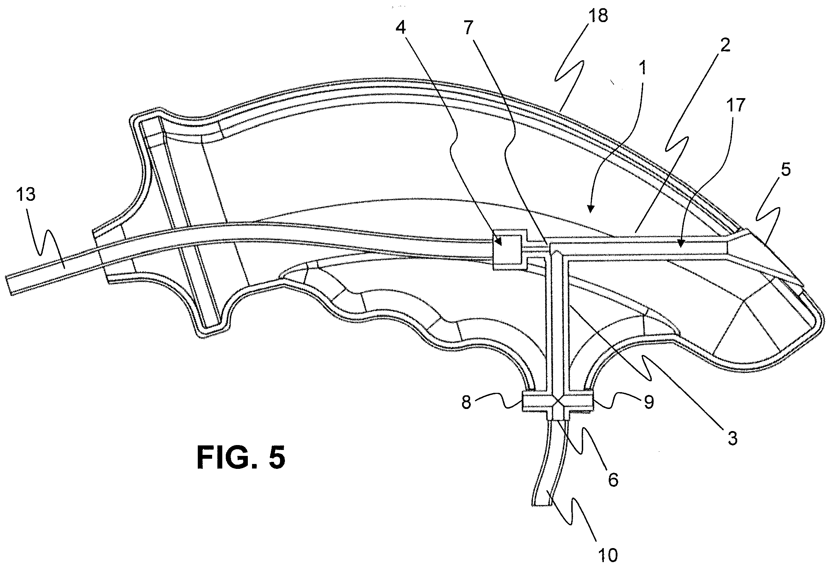

Figur 5 zeigt eine weitere Ausgestaltung des Gasstromumkehrelements 1, bei der das Gasstromumkehrelement 1 in einem ergonomisch geformten Gehäuse 18 angeordnet ist. Das Gehäuse 18 ist dabei so geformt, dass es an die menschliche Hand angepasst ist. Die Sicherheitsöffnungen 8, 9 lassen sich mit den Fingern, die Auslassöffnung 5 lässt sich mit dem Daumen ganz oder teilweise verschließen. Dabei spielt es keine Rolle, ob die Auslassöffnung 5, die Sicherheitsöffnungen 8, 9, der Druckanschluss 4 und/oder der Leitungsanschluss 6 unmittelbar an der Gehäuseaußenwand angebracht sind oder ob die jeweilige Verbindung zwischen Öffnung oder Anschluss und Gehäuseaußenwand durch ein Leitungsstück, beispielsweise einer Verbindungsleitung 13, realisiert wird. Durch eine derartige Ausgestaltung ist die Bedienung des Gasstromumkehrelements 1 mit nur einer Hand eines Helfers möglich. Zur einfacheren Handhabung kann auch nur eine Sicherheitsöffnung 8 vorgesehen werden. Das Gehäuse 18 und das Gasstromumkehrelement 1 können in verschiedener Weise zusammengesetzt oder zusammenhängend hergestellt werden. Wichtig ist, dass eine leicht manuell zu bedienende Vorrichtung entsteht, die in einer Hand gehalten werden kann, wobei die Auslassöffnung 5 und die Sicherheitsöffnungen 8, 9 mit den Fingern oder anderen Teilen dieser Hand verschlossen werden können.

Die vorliegende Erfindung ermöglicht eine effektive Beatmung eines Patienten. Unter Notfallbedingungen ist neben einem in den Atemweg eines Patienten einbringbaren Katheter 10 oder einer Kanüle und einer Verbindungsleitung 13 lediglich eine Druckgasquelle 11, z.B. eine Sauerstoffdruckflasche, notwendig. Hohe Sicherheit für Patient und Helfer ist bei dieser Beatmungstechnik gewährleistet. Auch eine Anwendung als Atemhilfe zur Unterstützung der Eigenatmung eines spontan atmenden Patienten ist möglich.

## Patentansprüche

1. Gasstromumkehrelement (1) zur Ausnutzung eines unter Überdruck stehenden Gasvorrates (14), insbesondere Sauerstoff, zur wahlweisen Erzeugung eines Gasstromes von oder zu einem Leitungsanschluss (6), der insbesondere mit einem Atemweg eines Patienten verbindbar ist, ausgebildet als ein Hauptstück (2) mit Abzweigungsstück (3), wobei das Hauptstück (2) einen Druckanschluss (4) zum Anschluss an den Gasvorrat (14) über einen Mischkanal (17) mit mindestens einer verschließbaren Auslassöffnung (5) verbindet und das Abzweigungsstück (3) das Hauptstück (2) mit dem Leitungsanschluss (6) verbindet, **dadurch gekennzeichnet, dass** in dem Hauptstück (2) der Mischkanal (17) und eine Düse (7), insbesondere eine Injektor-Düse, so ausgebildet und angeordnet sind, dass durch einen von dem Druckanschluss (4) durch die Düse (7) und durch den Mischkanal (17) zu der Auslassöffnung (5) strömenden Gasstrom **am Leitungsanschluss (6) ein Unterdruck entsteht und** im Hauptstück (2) bei geöffneter Auslassöffnung (5) auch ein Gasstrom im Abzweigungsstück (3) in Richtung Auslassöffnung (5) erzeugbar ist, und zwar mit einem Volumenstrom, der bei 1 bis 5 bar Überdruck über Umgebungsdruck am Druckanschluss (4) mindestens 8 Liter / Minute beträgt; **wobei bei geschlossener Auslassöffnung (5) am Leitungsanschluss (6) ein Überdruck entsteht und ein Gasstrom in Richtung Patient bewirkbar ist.**

2. Gasstromumkehrelement (1) nach Anspruch 1, **dadurch gekennzeichnet, dass** der Volumenstrom im Abzweigungsstück (3) mindestens das 0,4-fache, vorzugsweise das 0,6- bis 2-fache, des Volumenstroms durch den Druckanschluss (4) bei einem Überdruck von 1 bis 5 bar am Druckanschluss (4) beträgt.

3. Gasstromumkehrelement (1) nach Anspruch 1 oder 2, **dadurch gekennzeichnet, dass** die Länge (L2) des Mischkanals (17) und der Innendurchmesser (D1) der Düse (7) ein Verhältnis zwischen 2:1 und 100:1, bevorzugt zwischen 5:1 und 50:1 haben.

4. Gasstromumkehrelement (1) nach einem der Ansprüche 1 bis 3, **dadurch gekennzeichnet, dass** das Verhältnis (D2 : D3) des Innendurchmessers (D2) des Mischkanals (17) und des Innendurchmessers (D3) des Abzweigungsstücks (3) zwischen 1,5 und 0,5 liegt, vorzugsweise bei 1.

5. Gasstromumkehrelement (1) nach einem der Ansprüche 1 bis 4, **dadurch gekennzeichnet, dass** in dem Hauptstück (2) und/oder in dem Abzweigungsstück (3) mindestens eine Sicherheitsöffnung (8, 9) vorhanden ist, insbesondere aber zwei, bevorzugt zwei nebeneinander oder einander gegenüber liegende Sicherheitsöffnungen (8, 9) vorhanden sind.

6. Gasstromumkehrelement (1) nach Anspruch 5, **dadurch gekennzeichnet, dass** die Auslassöffnung (5) und die Sicherheitsöffnung(en) (8, 9) so angeordnet sind, dass sie manuell, vorzugsweise mit Handballen und/oder Fingern nur einer Hand eines Helfers wahlweise einzeln oder gemeinsam ganz oder teilweise verschließbar sind.

7. Gasstromumkehrelement (1) nach einem der Ansprüche 1 bis 5, **dadurch gekennzeichnet, dass** das Gasstromumkehrelement (1) in einem ergonomisch geformten Gehäuse angeordnet ist, das an die Hand eines Menschen angepasst ist, wobei die Auslassöffnung (5) und die mindestens eine Sicherheitsöffnung (8, 9) so angeordnet sind, dass sie manuell, vorzugsweise mit Handballen und/oder Fingern nur einer Hand eines Helfers wahlweise einzeln oder gemeinsam ganz oder teilweise verschließbar sind.

8. Gasstromumkehrelement (1) nach einem der vorhergehenden Ansprüche, **dadurch gekennzeichnet, dass** die Auslassöffnung (5) durch eine Lochscheibe (16) wechselweise ganz oder teilweise verschlossen und freigegeben wird.

9. Gasstromumkehrelement (1) nach einem der vorhergehenden Ansprüche, **dadurch gekennzeichnet, dass** der Druckanschluss (4) zur Herstellung einer Verbindung mit einer Druckgasquelle (11), vorzugsweise einer Sauerstoffdruckflasche, ausgebildet ist.

10. Gasstromumkehrelement (1) nach einem der vorhergehenden Ansprüche, **dadurch gekennzeichnet, dass** das Abzweigungsstück (3) einen verschließbaren Seitenzugang (12) aufweist, vorzugsweise zwischen der/den Sicherheitsöffnung(en) (8, 9) und dem Leitungsanschluss (6).

11. Gasstromumkehrelement (1) nach einem der vorhergehenden Ansprüche, **dadurch gekennzeichnet, dass** das Hauptstück (2) und das Abzweigungsstück (3) zusammen etwa ein T-Stück bilden mit dem Hauptstück (2) als Querbalken.

12. Gasstromumkehrelement (1) nach einem der vorhergehenden Ansprüche, **dadurch gekennzeichnet, dass** das Hauptstück (2) und das Abzweigungsstück (3) zusammen etwa ein Y-Stück bilden, wobei die Auslassöffnung (5) den Fuß des Y bildet.

13. Gasstromumkehrelement (1) nach einem der vorhergehenden Ansprüche, **dadurch gekennzeichnet, dass** das Gasstromumkehrelement (1) aus mehreren Teilelementen zusammengesetzt, insbesondere zusammengeschraubt ist.

14. Gasstromumkehrelement (1) nach einem der vorhergehenden Ansprüche, **dadurch gekennzeichnet, dass** der Druckanschluss (4) und/oder der Leitungsanschluss (6) und gegebenenfalls der Seitenzugang (12) als sogenannter "Luer Lock" ausgeführt sind.

15. Gasstromumkehrelement (1) nach einem der vorhergehenden Ansprüche, **dadurch gekennzeichnet, dass** das Gasstromumkehrelement (1) im Wesentlichen aus Kunststoff gefertigt ist, wobei die Düse (7) aus Kunststoff oder aus einem metallischen Einsatz besteht und das Hauptstück (2) einschließlich des Druckanschlusses (4) gekapselt ist.

## Claims

1. Gas flow reversing element (1) for the use of a gas supply (14) under excess pressure, in particular oxygen, for selectively generating a gas flow from or to a line connector (6) which can in particular be connected to an airway of a patient, said gas flow reversing element (1) being designed as a main piece (2) with a branching piece (3), the main piece (2) connecting a pressure connector (4), for connection to the gas supply (14), to at least one closable outlet opening (5) via a mixing channel (17), and the branching piece (3) connecting the main piece (2) to the line connector (6), **characterized in that** the mixing channel (17) and a nozzle (7), particularly an injector nozzle, are configured and arranged in the main piece (2) in such a way that, by a gas flow flowing from the pressure connector (4) through the nozzle (7) and through the mixing channel (17) to the outlet opening (5), an underpressure is generated at the line connector (6), whereas in the main piece (2), with the outlet opening (5) opened, a gas flow can also be generated in the branching piece (3) in the direction of the outlet opening (5), specifically with a volumetric flow rate which, at an excess pressure of 1 to 5 bar above atmospheric pressure at the pressure connector (4), is at least 8 liters per minute; wherein, with the outlet opening (5) closed an overpressure is generated at the line connector (6) and a gas flow in direction to the patient is achievable.

2. Gas flow reversing element (1) according to claim 1 , **characterized in that** the volumetric flow rate in the branching piece (3) is at least 0.4 times, preferably 0.6 to 2 times, the volumetric flow rate through the pressure connector (4) at an excess pressure of 1 to 5 bar at the pressure connector (4).

3. Gas flow reversing element (1) according to claim 2 or 3, **characterized in that** the length (L2) of the mixing channel (17) and the internal diameter (D1) of the nozzle (7) have a ratio of between 2:1 and 100:1, preferably of between 5:1 and 50:1.

4. Gas flow reversing element (1) according to one of claims 1 to 3, **characterized in that** the ratio (D2:D3) of the internal diameter (D2) of the mixing channel (17) and the internal diameter (D3) of the branching piece (3) is between 1.5 and 0.5, preferably 1.

5. Gas flow reversing element (1) according to one of claims 1 to 4, **characterized in that** at least one safety opening (8, 9) is present in the main piece (2) and/or in the branching piece (3), but in particular two of them, preferably two safety openings (8, 9) lying next to each other or opposite each other.

6. Gas flow reversing element (1) according to claim 5, **characterized in that** the outlet opening (5) and the safety opening(s) (8, 9) are arranged such that they can selectively be closed individually or together, completely or partially, for example manually, preferably with the ball of the thumb and and/or with the fingers of just one hand of a helper.

7. Gas flow reversing element (1) according to one of claims 1 to 5, **characterized in that** the gas flow reversing element (1) is arranged in an ergonomically shaped housing adapted to the human hand, the outlet opening (5) and the at least one safety opening (8, 9) being arranged such that they can selectively be closed individually or together, completely or partially, for example manually, preferably with the ball of the thumb and and/or with the fingers of just one hand of a helper.

8. Gas flow reversing element (1) according to one of the preceding claims, **characterized in that** the outlet opening (5) is alternately closed and opened, completely or partially, by an apertured disk (16).

9. Gas flow reversing element (1) according to one of the preceding claims, **characterized in that** the pressure connector (4) is designed to establish a connection to a compressed gas source (11), preferably a compressed oxygen cylinder.

10. Gas flow reversing element (1) according to one of the preceding claims, **characterized in that** the branching piece (3) has a closable side access (12), preferably between the safety opening(s) (8, 9) and the line connector (6).

11. Gas flow reversing element (1) according to one of the preceding claims, **characterized in that** the main piece (2) and the branching piece (3) together form approximately a T-piece, with the main piece (2) as crossbeam.

12. Gas flow reversing element (1) according to one of the preceding claims, **characterized in that** the main piece (2) and the branching piece (3) together form approximately a Y-piece, with the outlet opening (5) forming the foot of the Y.

13. Gas flow reversing element (1) according to one of the preceding claims, **characterized in that** the gas flow reversing element (1) is assembled, in particular screwed together, from several component parts.

14. Gas flow reversing element (1) according to one of the preceding claims, **characterized in that** the pressure connector (4) and/or the line connector (6), and if appropriate the side access (12), are designed as a Luer lock.

15. Gas flow reversing element (1) according to one of the preceding claims, **characterized in that** the gas flow reversing element (1) is made substantially of plastic, the nozzle (7) being composed of plastic or of a metal insert, and the main piece (2) including pressure connector (4) is encapsulated.

## Revendications

1. Elément (1) d'inversion du sens d'écoulement d'un gaz destiné à utiliser une réserve (14) de gaz, en particulier d'oxygène, placée en surpression, en vue de produire sélectivement un écoulement de gaz depuis un raccordement (6) de conduit ou vers celui-ci, lequel peut être être relié en particulier à une voie respiratoire d'un patient et configuré comme pièce principale (2) dotée d'une pièce de dérivation (3), la pièce principale (2) reliant un raccordement (4) sous pression, destiné à être raccordé à la réserve (14) de gaz par un canal de mélange (17), à au moins une ouverture (5) de sortie apte à être refermée, et la pièce de dérivation (3) reliant la pièce principale (2) au raccordement (6) de conduit, **caractérisé en ce que** le canal de mélange (17) et une tuyère (7), en particulier une tuyère à injecteur, sont configurés et disposés dans la pièce principale (2) de telle sorte qu'une dépression soit occasionnée au niveau du raccordement (6) de conduit par le biais d'un écoulement de gaz qui s'écoule du raccordement (4) sous pression à l'ouverture de sortie (5) en passant par la tuyère (7) et par le canal de mélange (17) et que, dans la pièce principale (2), lorsque l'ouverture de sortie (5) est ouverte, un écoulement de gaz puisse également être produit dans la pièce de dérivation (3) en direction de l'ouverture de sortie (5), et ce avec un débit volumique qui représente au moins 8 litres/minute pour une surpression de 1 à 5 bars au-dessus de la pression environnante au niveau du raccordement (4) sous pression ; une surpression se produisant et un écoulement de gaz dans la direction du patient pouvant être provoqué lorsque l'ouverture de sortie (5) au niveau du raccordement (6) de conduit est fermée.

2. Elément (1) d'inversion du sens d'écoulement d'un gaz selon la revendication 1, **caractérisé en ce que** le débit volumique dans la pièce de dérivation (3) vaut au moins 0,4 fois, de préférence 0,6 à 2 fois le débit volumique à travers le raccordement (4) sous pression pour une surpression de 1 à 5 bars au niveau du raccordement (4) sous pression.

3. Elément (1) d'inversion du sens d'écoulement d'un gaz selon la revendication 1 ou 2, **caractérisé en ce que** la longueur (L2) du canal de mélange (17) et le diamètre intérieur (D1) de la tuyère (7) présentent un rapport compris entre 2:1 et 100:1, de préférence entre 5:1 et 50:1.

4. Elément (1) d'inversion du sens d'écoulement d'un gaz selon l'une quelconque des revendications 1 à 3, **caractérisé en ce que** le rapport (D2 : D3) du diamètre intérieur (D2) du canal de mélange (17) au diamètre intérieur (D3) de la pièce de dérivation (3) est compris entre 1,5 et 0,5, de préférence vaut environ 1.

5. Elément (1) d'inversion du sens d'écoulement d'un gaz selon l'une quelconque des revendications 1 à 4, **caractérisé en ce qu'**au moins une ouverture de sécurité (8, 9), mais en particulier deux ouvertures de sécurité, de préférence deux ouvertures de sécurité (8, 9) disposées l'une à côté de l'autre ou l'une en face de l'autre, sont prévues dans la pièce principale (2) et/ou dans la pièce de dérivation (3).

6. Elément (1) d'inversion du sens d'écoulement d'un gaz selon la revendication 5, **caractérisé en ce que** l'ouverture de sortie (5) et la ou les ouvertures de sécurité (8, 9) sont disposées de telle sorte qu'elles puissent être fermées manuellement, de préférence à l'aide de la paume de la main et/ou des doigts d'une seule main d'un opérateur, de manière sélective séparément ou ensemble, et ce totalement ou partiellement.

7. Elément (1) d'inversion du sens d'écoulement d'un gaz selon l'une des revendications 1 à 5, **caractérisé en ce que** l'élément (1) d'inversion du sens d'écoulement de gaz est disposé dans un boîtier de forme ergonomique qui est adapté à la main d'un être humain, l'ouverture de sortie (5) et la ou les ouvertures de sécurité (8, 9) étant disposées de telle sorte qu'elles puissent être fermées manuellement, de préférence à l'aide de la paume de la main et/ou des doigts d'une seule main d'un opérateur, de manière sélective séparément ou ensemble, et ce totalement ou partiellement.

8. Elément (1) d'inversion du sens d'écoulement d'un gaz selon l'une des revendications précédentes, **caractérisé en ce que** l'ouverture de sortie (5) peut être fermée ou libérée totalement ou en partie par une plaque perforée (16).

9. Elément (1) d'inversion du sens d'écoulement d'un gaz selon l'une des revendications précédentes, **caractérisé en ce que** le raccordement (4) sous pression est configuré pour établir une liaison avec une source (11) de gaz sous pression, de préférence une bouteille d'oxygène sous pression.

10. Elément (1) d'inversion du sens d'écoulement d'un gaz selon l'une des revendications précédentes, **caractérisé en ce que** la pièce de dérivation (3) présente un accès latéral (12) apte à être refermé situé de préférence entre la ou les ouvertures de sécurité (8, 9) et le raccordement (6) de conduit.

11. Elément (1) d'inversion du sens d'écoulement d'un gaz selon l'une des revendications précédentes, **caractérisé en ce que** la pièce principale (2) et la pièce de dérivation (3) forment ensemble une pièce en T, la pièce principale (2) formant la branche transversale.

12. Elément (1) d'inversion du sens d'écoulement d'un gaz selon l'une des revendications précédentes, **caractérisé en ce que** la pièce principale (2) et la pièce de dérivation (3) forment ensemble une pièce en forme de Y, l'ouverture de sortie (5) formant le pied du Y.

13. Elément (1) d'inversion du sens d'écoulement d'un gaz selon l'une des revendications précédentes, **caractérisé en ce que** l'élément (1) d'inversion du sens d'écoulement de gaz est constitué de l'assemblage de plusieurs éléments partiels, en particulier vissés.

14. Elément (1) d'inversion du sens d'écoulement d'un gaz selon l'une des revendications précédentes, **caractérisé en ce que** le raccordement (4) sous pression et/ou le raccordement (6) de conduit et éventuellement l'accès latéral (12) sont configurés comme raccords dits "Luer Lock".

15. Elément (1) d'inversion du sens d'écoulement d'un gaz selon l'une des revendications précédentes, **caractérisé en ce que** l'élément (1) d'insertion du sens d'écoulement de gaz est réalisé essentiellement en matière synthétique, la tuyère (7) étant constituée de matière synthétique ou d'une garniture métallique et la pièce principale (2), y compris le raccordement (4) sous pression, étant encapsulée.
